# EUROPEAN PATENT APPLICATION

(11) **EP 1 224 943 A1**
(43) Date of publication of application: **24.07.2002**
(21) Application number: 01200212.7
(22) Date of filing: 19.01.2001
(51) Int. Cl.: A61K 39/395, C07K 16/28, A61P 35/00, A61K 47/48, A61K 51/10, G01N 33/53, C12N 5/10, C12N 15/13, C07K 16/18, C12N 15/62, A01K 67/027, A01H 5/00, C07K 14/78

(54) **Fibronectin as a tumor marker detected by phage antibodies**

(71) Applicant: Crucell Holland B.V., 2333 CN Leiden (NL)
(72) Inventor: The designation of the inventor has not yet been filed
(74) Representative: Prins, Adrianus Willem

(57) **Abstract**

Means and method for at least partial inhibition of tumor growth are provided. Methods makes use of binding molecules capable of specifically binding to an epitope present on a subset of fibronectin proteins. By providing an individual with a binding molecule of the invention it is possible to interfere with sites of angiogenesis or sites that seen active angiogenesis in the recent past. Through this interference blood flow is at least in part inhibited. Through this inhibition it is possible to at least in part inhibit processes dependent on active angiogenesis in said individual, such as tumor growth.

## Description

The invention relates to the field of medicine, more specifically to the treatment of cancer. The invention relates to antibodies, and particularly to antibodies that discriminate between human fibronectin present in normal tissue and in tissue containing tumor cells. Fibronectin can now be used as marker of tumor cell versus healthy cells and as a marker for angiogenesis.

Worldwide, much research has been done on cancer therapy. Success has been booked using chemotoxic medicaments and irradiation procedures. Often these treatments are specifically directed towards fast-replicating cells, because tumor cells are often replicating in a fast and uncontrolled way. However, these treatments often also affect other replicating cells, like hair cells, nail cells and sex cells. Therefore, these treatments are not exclusively tumor-cell specific, leading to damage to normal cells. This may result in dysfunction and/or elimination of normal cells. Patients may then suffer from side effects, for instance nausea and extreme fatigue. Moreover, said treatments are not always able to eliminate all tumor cells present in a patient. Only a part of patients suffering from cancer can be cured, depending on the kind of cancer. Furthermore, the effectiveness of a treatment varies between patients.

Presently, much effort is being done on cell-specific gene therapy. Using a gene delivery vehicle with tropism for tumor cells, it would be possible to specifically deliver a therapeutic gene of interest to a tumor cell, leaving other, non-tumor cells unaffected. A gene of interest to be delivered to a tumor cell may for instance be a gene encoding a protein whose expression leads to apoptosis or a protein that kills the cell and destroys other surrounding cells by its by-stander effect.

Another way of targeting tumor cells specifically is by the use of monoclonal antibodies that are directed against certain tumor specific antigens.

B-lymphocytes can produce antibodies in response to exposure to biological substances like bacteria, viruses and their toxic products. Antibodies are generally epitope specific and bind strongly to said biological substances carrying these epitopes. The hybridoma technique (Köhler and Milstein 1975) makes use of the ability of the B cells to produce monoclonal antibodies to specific antigens and to subsequently isolate and produce monoclonal antibodies by fusing B cells from mice exposed to the antigen of interest to immortalized murine plasma cells. This technology resulted in the realization that monoclonal antibodies produced by hybridoma's could be used in research, diagnostics and therapies to treat different kinds of diseases like cancer and auto-immune related disorders.

Because antibodies that were produced in mouse hybridoma's induced dramatic immune responses in humans, it became clear that the antibodies that were required for successful treatments of diseases in human needed to fit the human requirements to lower these immune responses. For this, murine antibodies were first engineered by replacing the murine constant domains with human constant regions. Subsequently, domains between the variable domains, that specify the antigen binding were replaced by their human counterparts. The final stage in this humanization process is the production of fully human antibodies. To date many different diseases are being treated with either humanized or fully human antibodies. Nevertheless, many disorders are not being treated since no specific epitopes are found, that are expressed on cells or substances that need to be removed by the immune system through the interaction with antibodies or other proteinaceous molecules capable of binding said epitope.

The present invention provides among others novel fibronectin associated epitopes, methods for finding these epitopes and proteinaceous molecules capable of binding to said novel epitopes. In one aspect the invention provides the extracellular matrix as a location were novel fibronectin associated epitopes can be found. The extra-cellular matrix controls cell survival, cell morphology and tissue organization by supporting cell adhesion. Remodeling of the extra-cellular matrix is a key process in the development of properly organized blood vessels, tissues and organs. Many extra-cellular matrix proteins, such as fibronectin and members of the collagen family, and proteases, such as plasmin and metalloproteinases, are involved in the remodeling process. At the cellular level, remodeling is regulated by members of the integrin family of adhesion receptors, which use the extracellular matrix proteins as attachment sites for migration.

A large number of studies have indicated that matrix-remodeling and cell-migration also play a key role during pathological processes (Werb 1997; Liotta et al. 1991). For example, proteases including the matrix metalloproteinases (MMPs) are understood to not only facilitate migration of cells by degradation of matrices, but also to affect tumor formation and growth. Inhibition of proteases, such as urokinase-type plasminogen activator (uPA) or MMPs prevent the formation of metastases (Jankun et al. 1997; Min et al. 1996; Ignar et al. 1998). Cell interactions with extracellular matrices are important to pathological changes that occur during cell transformation and tumorigenesis. Several extracellular matrix proteins including fibronectin, thrombospondin-1, laminin, SPARC, and osteopontin have been suggested to modulate tumor phenotype by affecting cell migration or survival, while matrix-remodeling and integrin dependent migration is also crucial for angiogenesis, which is the formation of new blood vessels (Friedlander et al. 1995). The integrins αvβ3 and αvβ5 in particular play an important role during this angiogenesis process (Brooks et al. 1994). Their expression is elevated on angiogenic endothelial cells and antagonists of αvβ3 suppress tumor growth and can induce tumor regression.

Fibronectin, which is one of the proteins that is suggested to play a role in the modulation of tumor phenotypes, is a high molecular glycoprotein expressed at the cell surface of many types of differentiated cells and is involved in the attachment of cells to the surrounding extracellular matrix. Fibronectin has affinity to the other main components of extracellular matrix, collagen and glycosaminoglycans. It also interacts with cell surfaces as shown by the finding that fibronectin-collagen complexes, or fibronectin alone when insolubilized on a surface such as plastic, enhances the attachment of various types of cells to such surfaces.
Fibronectin is a dimer and consists of large disulfide-linked subunits composed of multiple structural domains. More than half of the molecule consists of so-called fibronectin type III repeats.

Two splice-variant of fibronectin are known. A so-called ED-A splice variant is expressed in some normal tissues and can also be found in the serum. ED-A expression is upregulated in tumor tissue and embryonic tissue. A so-called ED-B splice variant is only expressed in embryonic tissue and tumor tissue. It is not detectable in healthy adult tissue (Reza Farmould et al. 1995; Pujuguet et al. 1996).

In one aspect the invention provides a novel epitope on fibronectin. The epitope is different from the ED-A en ED-B epitopes described earlier. The expression pattern throughout the body is not reminiscent of either of these described epitopes. The observed expression pattern of the epitope of the invention in a human body is unique and offers a new way of treatment of disease. Expression of an epitope of the invention is associated with at least some kinds of tumor cells, some kinds of endothelial cells in the vicinity of tumor cells and extra-cellular matrix in the vicinity of tumor cells. It is possible that an epitope of the invention is correlated with area's of active angiogenesis or area's where angiogenesis has occurred in the recent past.

In another aspect the invention provides a proteinaceous molecule capable of specifically binding to an epitope present in a subset of fibronectin proteins. Preferably, said epitope is present in a subset of cells expressing fibronectin. In one embodiment an epitope of the invention is a conformational epitope. An epitope of the invention is not expressed in any of the tested normal human tissues. It is however, expressed on some types of tumor cells, some kinds of endothelial cells in the vicinity of tumor cells and extra-cellular matrix in the vicinity of tumor cells. In a preferred embodiment therefore said proteinaceous molecule is capable of distinguishing a subset of fibronectin containing matrices and/or fibronectin containing cells. Binding of a proteinaceous molecule capable of specifically binding to an epitope present in a subset of fibronectin proteins can be used in a diagnostic setting. For instance, to determine whether tumor tissue is present in a sample taken from a patient. The binding of said proteinaceous molecule to its target can also be used in therapeutic settings. In a particularly preferred embodiment the invention provides a method for at least in part preventing growth of tumor cells comprising providing said tumor cells and/or surrounding matrix tissue with a proteinaceous molecule capable of specifically binding to an epitope present in a subset of fibronectin proteins. Tumor growth is at least in part prevented through direct interaction of said proteinaceous molecule with said subset of fibronectin proteins. Fibronectin not belonging to the subset is not bound by said proteinaceous molecule and therefor not subject to the effects of the proteinaceous molecule. Without being bound by theory it is believed that binding of said proteinaceous molecule interferes with one or more signaling properties of fibronectin or fibronectin-associated molecules. Fibronectin, especially in provisional extracellular matrix in area's where blood vessel formation occurs, facilitates such blood vessel formation. This facilitation can at least in part be interfered with, by allowing a proteinaceous molecule of the invention to bind to said fibronectin. Preferably, the epitope for binding of the proteinaceous molecule is at least in part specific for the tissue where blood vessel formation is to be at least in part inhibited. Preferably, said epitope comprises ED-A or ED-B. More preferably, said epitope comprises an epitope of the invention. The distribution of an epitope of the invention is particularly favorable for use in therapeutic settings not in the least because it has not been detected in any of the normal tissues tested yet. By providing an individual with a binding molecule of the invention it is possible to interfere with sites of angiogenesis or sites that seen active angiogenesis in the recent past. Through this interference blood flow is at least in part inhibited. Through this inhibition it is possible to at least in part inhibit processes dependent on active angiogenesis in said individual, such as tumor growth. The absence of an epitope of the invention in normal tissue allows systemic administration routes for the proteinaceous molecule of the invention. This is advantageous because it allows the use of the blood stream for dissemination of the proteinaceous molecule of the invention, with all the associated advantages (suitable penetration and distribution). A limited availability of the targeted epitope outside the area targeted with the therapy is usually tolerated for two reasons. Blood vessel formation can be inhibited in a normal individual, for at least a limited period, without invoking serious side effects. Individuals in which angiogenesis should not be interfered with, such as pregnant women or women that want to conceive, might observe miscellaneous detrimental effects. If need be, patients in which side effects are anticipated can be excluded from receiving a fibronectin binding proteinaceous molecule of the invention.

In some embodiments of the invention the binding of a proteinaceous molecule of the invention can recruit immune system components to bound fibronectin. This recruitment facilitates removal of the bound extracellular matrix and/or the bound cell. Alternatively, said proteinaceous molecule may comprise or be provided with a tag. Preferably said tag is capable of interfering with cellular processes. With said preferred tag said proteinaceous molecule is capable of at least in part improving the anti-tumor capabilities of a proteinaceous molecule of the invention. Preferably, said tag comprises an angiogenesis inhibitor, a toxin, a cytostatic drug and/or a radioactive compound.

In a preferred aspect of the invention, said proteinaceous molecule is capable of binding to a neoplastic cell. Such binding can be used to mark said cell in a diagnostic assay or in a therapeutic setting. For instance, binding of a proteinacous molecule of the invention may trigger the death of the bound cell. More preferably, said neoplastic cell is derived from a breast cell, prostate cell, colon cell, lung cell, skin cell, pancreas cell, bladder cell, head cell or a neck cell. In another preferred embodiment said proteinaceous molecule is capable of binding to a microvascular endothelial cell. Preferably, said microvascular cell lies in the vicinity of a tumor cell. Binding of a proteinaceous molecule of the invention to a microvascular cell can be used to at least in part prevent outgrowth of a new blood vessel thereby limiting the blood supply to tumor cells thereby at least in part limiting the growth of said tumor cells. An epitope of the invention is present in different types of animals. However, considering the clinical and diagnostic uses said proteinaceous molecule is preferably capable of binding to a subset of human fibronectin proteins.

Many different types of binding molecules are known in the art. For proteinaceous targets such as fibronectin, binding molecules are typically proteinaceous molecules. They at least comprise some amino-acids linked through a peptide linkage. Typically such proteinaceous binding molecules comprise at least 7 amino-acids that are linked through peptide linkage. A proteinaceous molecule of the invention can be found for instance using phage display of peptides. In a preferred embodiment of the invention said proteinaceous molecule is an antibody or a functional part or derivative thereof. A functional part of an antibody comprises at least one antigen binding part of said antibody. Non-limiting parts are FAB 1 and FAB 2 fragments. A derivative or a part of an antibody comprises essentially the same antigen binding properties of a proteinaceous molecule of the invention in kind not necessarily in amount. A derivative may comprise one or more amino-acid substitutions, insertions or deletions relative to an antibody or part thereof, of the invention. In another preferred embodiment said proteinaceous molecule is a single chain antibody. Preferably, said antibody or functional part, or derivative thereof is human or humanized. Preferably, a proteinaceous molecule of the invention further comprises a tag. Preferably, said tag comprises a toxin and/or a radioactive substance. In a preferred embodiment said proteinaceous molecule comprises a heavy chain comprising a CDR3 region, said CDR3 region comprising the sequence EDTAVYYCAR NPFQSS FDYWGQ or a derivative and/or functional analogue thereof. In another preferred embodiment said proteinaceous molecule comprises a light chain comprising a CDR3 region, said CDR3 region comprising the sequence EDFATYYC SQFSTMPGGFGQGTK VEIK or a derivative and/or functional analogue thereof. A derivative and/or functional analogue of a heavy chain or a light chain mentioned above is a molecule comprising the same heavy or light chain activities in kind not necessarily in amount. Said heavy or light chain activities preferably include antigen-binding activities of the one or the combination of the chains.

In one aspect the invention provides a method for the treatment of an individual suffering from or at risk of suffering from a disease, comprising administering to said individual a therapeutically acceptable amount of a proteinaceous molecule of the invention. Preferably, said disease is a neoplastic disease. In a similar aspect the invention provides the use of a proteinaceous molecule of the invention for the preparation of a medicament. In yet another embodiment the invention provides a method for typing a cell comprising determining whether said cell is capable of specifically binding a proteinaceous molecule of the invention. Similarly the invention also provides the use of an epitope expressed on a subset of fibronectin expressing cells as a marker for neoplastic cells.

With the current technology it is possible to obtain nucleic acid encoding a proteinaceous binding molecule. Particularly for peptides and antibody like binding molecules it is possible to obtain encoding nucleic acid. This can be done for instance through amplifying the selected phages in phage display approaches or the amplification of antibody encoding RNA in hybridoma approaches. The invention therefor further provides a nucleic acid encoding a proteinaceous molecule of the invention, or a part thereof involved in fibronectin binding of said molecule. For production of proteinaceous molecule of the invention encoding nucleic acid can be cloned into one or more suitable expression vectors and transferred to cells. The invention therefor also provides a cell comprising a nucleic acid of the invention. Preferably, said cell is a primate, rodent, bird or plant cell. Such cells are particularly suited for the production of binding molecules. Preferably, said cell is a human cell. In this way the binding molecule is produced by a human cell and should therefor comprise post-translational modifications that are compatible with use in humans. It has been observed that such binding molecules have improved pharmaco-dynamic properties in humans. Preferably, said expression vector comprises means for the conditional expression of a nucleic acid of interest. The invention thereof further provides a cell comprising a means for the conditional expression of a nucleic acid of interest. Preferably, said means comprises a tetracycline responsive expression system. It yet another preferred embodiment said cell further comprises nucleic acid encoding an early protein of adenovirus or a functional part, derivative and/or analogue thereof. Such cells are typically very well suited for obtaining high yields of protein. Preferably, said early protein comprises adenovirus E1 or a functional part, derivative and/or analogue thereof. In another preferred embodiment said cell comprises adenovirus E2A or a functional part, derivative and/or analogue thereof. Such cells typically show improved culture characteristics such as suspension growth and serum free adaptation.

In the field binding molecules are also produced using a plant or a non-human animal. The invention therefor also provides such production platforms. In a preferred embodiment said plant or non-human animal is transgenic for a nucleic acid of the invention.

In yet another aspect the invention provides a gene delivery vehicle comprising a proteinaceous molecule of the invention. Such gene delivery vehicles are targeted toward said subset of fibronectin proteins. Such a gene delivery vehicle can be used in a method for the treatment of an individual suffering from or at risk of suffering from a disease, comprising administering to said individual a therapeutically acceptable amount of a gene delivery vehicle of the invention.
Preferably said disease is a neoplastic disease. A gene delivery vehicle of the invention can favorably be used for the preparation of a medicament.

Provides is further a kit comprising a proteinaceous molecule and/or a gene delivery vehicle of the invention.

### Examples

### Example 1: Phage selection on Human Microvascular Endothelial cells (HDMEC-1)

T75 flasks, T175 flasks and 6 well plates were coated by overlaying the bottom overnight with a 1% gelatine/PBS solution. The flasks were then washed three times with PBS and stored with 10 ml PBS at 4°C. Endothelial cells were obtained from the Center for Disease Control and prevention (CDC, Atlanta, USA. Ades et al. 1992). The cells were grown on the gelatin coated T75 flasks in Dulbecco's Modified Eagle Medium (DMEM) with sodium pyruvate, 100mM glucose and pyridoxine (Gibco) supplemented with 20% Human Pooled Serum (HPS) made from healthy donors, penicilline and streptomycin. The cells were further cultured in an incubator at 37°C, 5% CO₂.
The Endothelial cells were grown to 80-90% confluency in DMEM + 20% HPS + pen/strep in a gelatine coated T75 flask and subsequently treated with trypsin in order to make a single cell suspension, washed with PBS and seeded in a 6 well plate at 20-30% confluency. Normally cells reached 65-70% confluency after 2 days of culturing and subsequently used for the phage selection procedure.

The scFv antibody phage display library used for selection procedures, is a semi-synthetic scFv library with a synthetic CDR3 region in the variable heavy chain derived from a semi-synthetic scFv library described by De Kruif et al. (1995). 500 µl of the scFv antibody phage library was incubated with 1.5 ml PBS containing 4% non-fat milk powder (Protifar), 1% gelatine, 20% HPS and 5x10⁸ Periferal Blood Cells (PBL's) for 30 minutes at 4°C. The Endothelial cells were then washed 6 times with 3 ml PBS at 4°C and incubated with the previously described library mix for 4 hrs at 4°C while shaking gently on a rocking platform. After this incubation and phage binding period, cells were washed extensively (20 times) with PBS + 0.01% Tween20 and 20 times with PBS at 4°C. The Endothelial cells were subsequently harvested using a cell scraper and spun down at 1500 rpm. The cells and the phages bound to them were then incubated with 10 ml of a fresh XL1-blue bacteria culture (Stratagene) at OD₆₀₀ 0.5 and left at 37 °C for 30 min for infection of the bacteria by the phages. Bacteria were harvested by spinning at 3600 rpm and plated on TYE dishes (10 g/l Bacto-tryptone, 5 g/l yeast extract, 15 g/l bactoagar (Gibco) and 8 g/l NaCl (Riedel-deHaën)) containing 100 µg/ml ampicilline and 10 µ g/ml tetracycline. Bacterial colonies were counted,scraped from the plates and used as inoculum for the next round of phage rescue according to Marks et al. (1991).

### Example 2: Analysis of isolated phage

Monoclonal phages were isolated according to Marks et al. (1991) and isolated phages were tested for binding using flow cytometry analysis on Human Microvascular Endothelial cells, that were used for the selection procedure (HDMEC-1). For this, HDMEC-1 cells were detached from the flasks by incubating the cells in 50 mM EDTA in PBS at 4°C for 15 min. Cells were then incubated with 100 µl pre-blocked phage maxiprep (50 µl phage prep was incubated for 15 min at 4°C with 50 µl PBS 4% milk powder (Protifar) for 30 min at 4°C. The cells were then washed 4 times with 200 µl PBS 1% BSA and incubated for 30 min at 4°C with Mouse anti M13 (Amersham) that was diluted 1:500 in PBS 1% BSA. After 4 times washing the cells were incubated with Goat anti Mouse PE 1:500 (Southern Biotechnology Associates, Inc) for 30 min at 4°C. After 4 times washing with PBS 1% BSA cells were analyzed on a FACS calibur for positive binding of the phages to the HDMEC cell line that was used for the selection procedure.

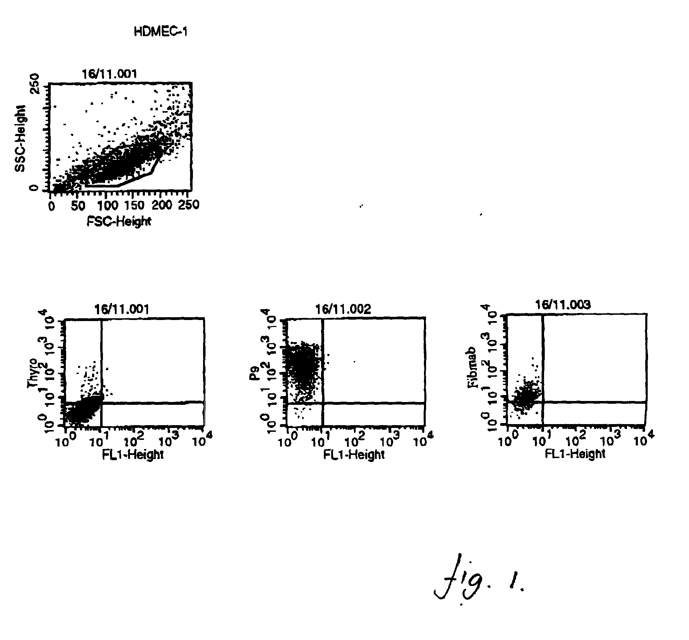

Fig.1 shows the specific FACS staining of HDMEC-1 cells that were incubated with the isolated phage. The single phage that was isolated and that carries a scFv that recognizes HDMEC-1 cells specifically was named FibMab. The P9 scFv served as a positive control since it was identified in the same screen as FibMab for binding to the HDMEC-1 cells and the Thyro scFv (identical to the Tg scFv) served as a negative control (see below).

### Example 3: Analysis of FibMab using a large panel of different cell lines.

FibMab was analyzed on a large panel of cell lines and Peripheral Blood Leucocytes using the same staining protocol as described before. A positive score (depicted as +) was determined by a staining intensity that was higher than that of a negative phage staining. Results are shown in Table I.

**Table I.**

| Cells that were tested for binding by FibMab. | | | | | |
|---|---|---|---|---|---|
| cell line | score | cell line | score | cell line | score |
| Ls 174 T | - | RPMI 8226 | + | HDMEC-1 | + |
| Ls 180 | - | CEM | - | UM26 | - |
| UM 9 | - | HELA | - | HUVEC | - |
| UM 3 | - | HepGII | - | ECL-4 | - |
| Daudi | - | BHK | - | U266 | - |
| Raji | - | COS | - | DOX 40 | +/- |
| HL60 | - | K563 | - | HT29 | - |
| Fravel | - | THP1 | - | U937 | - |
| Jurkat | - | 293T | - | | |

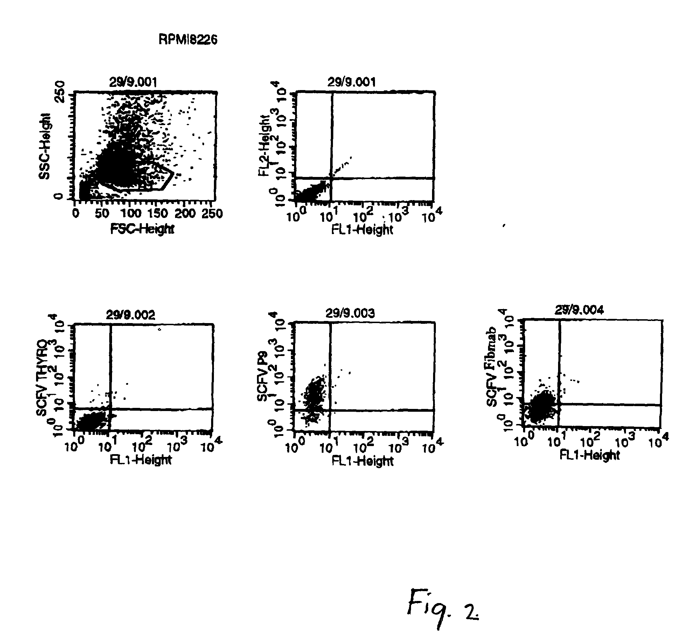

The results depicted in Table I show that FibMab recognizes an epitope present on three cell lines: The formerly used cells HDMEC-1, the plasma cell line RPMI 8226 and the plasma cell line DOX 40, which gave a slightly less intense staining as compared to HDMEC-1 and RPMI 8226. DOX 40 is a doxorubicine resistant variant of the cell line RPMI 8226. Fig.2 shows the FACS analysis of FibMab on the plasma cell line RPMI 8226.

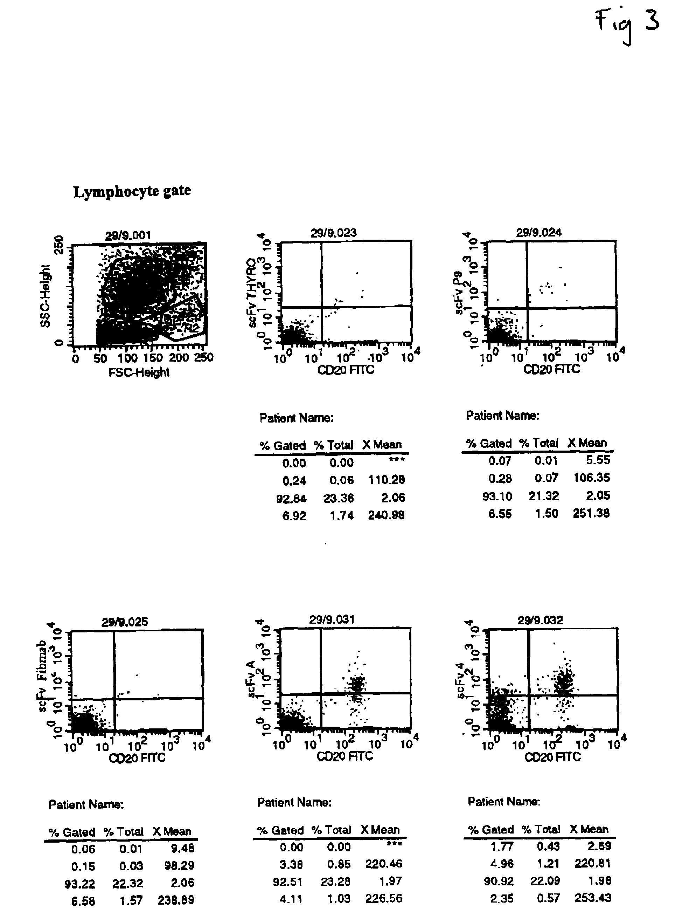

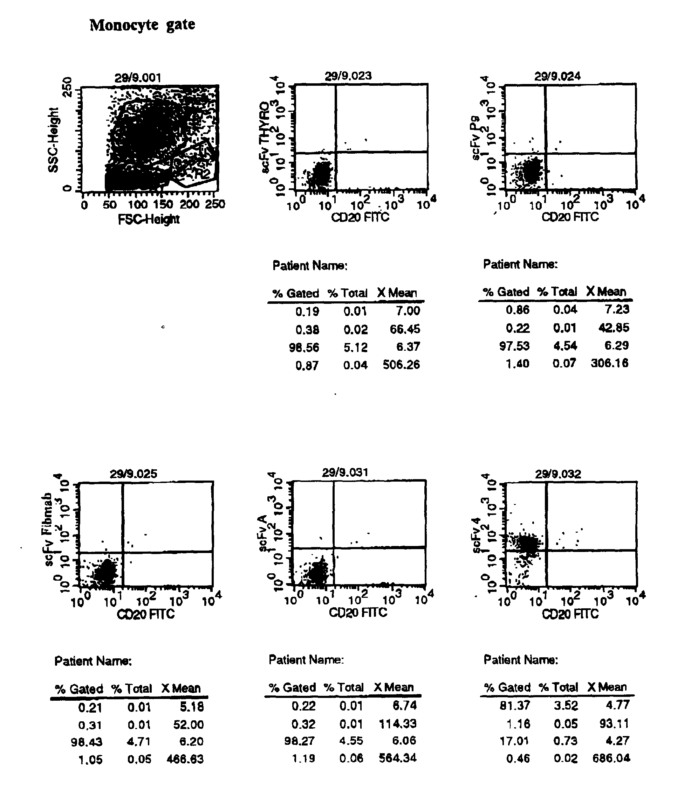

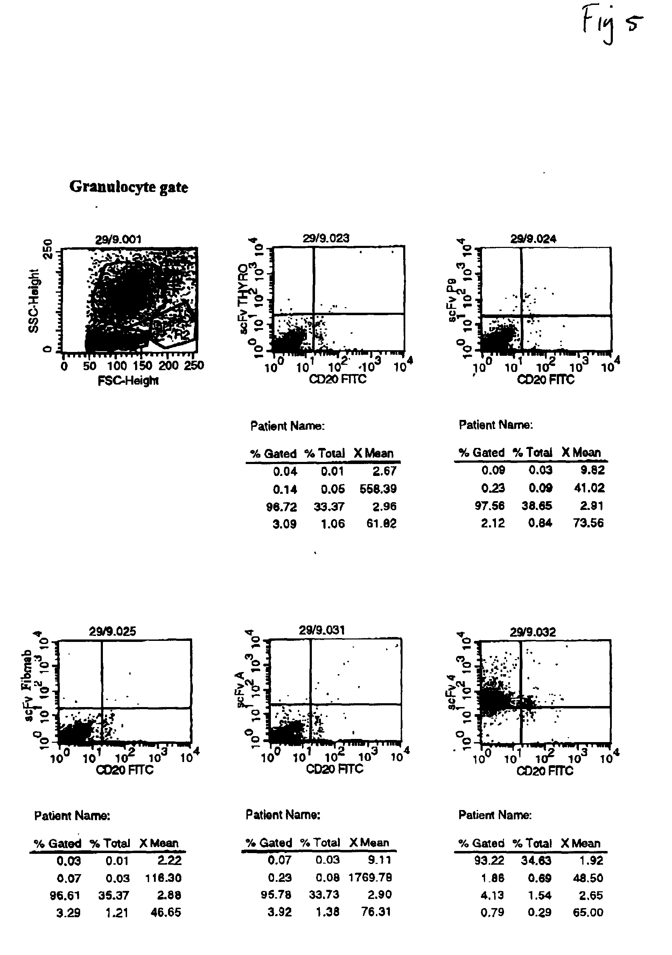

Fig.3,4 and 5 show the FACS analyses of FibMab on different compartments of the tested peripheral blood leucocytes (PBL's) as indicated in the short descriptions. Thyro (Tg) and P9 scFv's served as negative and positive controls respectively (see above) .

### Example 4: Determination of heavy and light chain variable region sequences present in FibMab.

The nucleotide sequences of the variable heavy chain region (Vh) and the variable light chain region (Vl) were determined using the ABI373 Xl system (Applied Biosystems) following the instructions given by the manufacturer. For this, 8 µl of big dye terminator mix, 3.2 pmol of primer (diluted in 1 µl), 5 µl plasmid miniprep (Qiagen) and 6 µl of bidest were mixed and incubated in a thermocycler (Rocidile III, Apligen). The PCR program that was used was: 96°C 1 min followed by 25 times three steps: 1) 96°C for 10 sec, 2) 50°C for 5 sec and 60°C for 4 min. The reactions were then cooled to 4°C and analyzed by the ABI 373 XL system. From the nucleotide sequence, the amino acid sequence of the heavy (Vh) and light (Vl) chain and the synthetic hypervariabel CDR3 region (binding domain of and antibody) were determined. Reagents from the Big Dye terminating kit (PE) were applied according to standard protocols well known to persons skilled in the art. Standard FDseq and M13R sequence primers were used. Identified sequences are given in Table II.

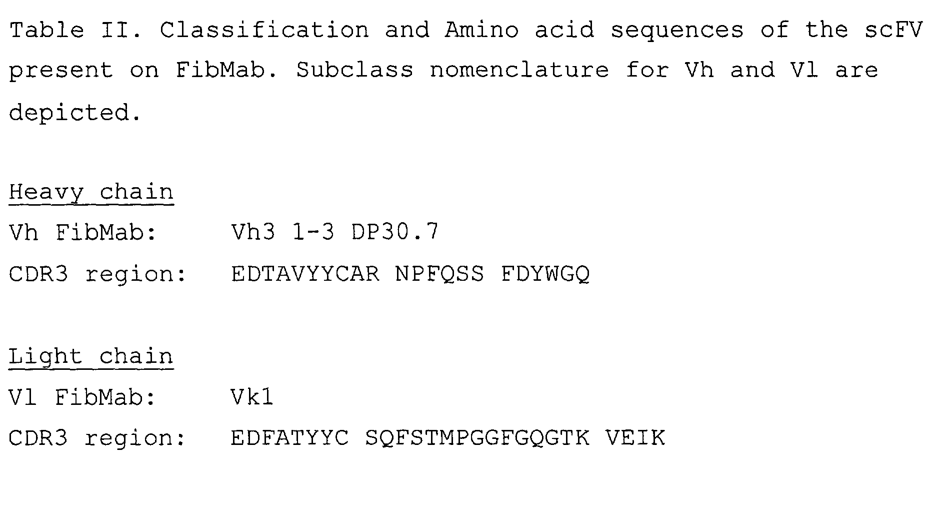

### Example 5: Immuno histochemical analysis of the scFv from phage FibMab on Grawitz tumor cells and healthy kidney tissue.

For the preparation of scFv preps, the DNA encoding the scFv was cloned into a His-Myc construct by digesting the pPVT plasmid of FibMab with *NcoI* and *NotI* to excise the Vh and Vl of the scFv of FibMab and ligating this insert into a pPVT construct containing both a Histidine-taq and a Myc-taq for detection and purification, resulting in the construct FibMabHisMyc. scFv (TES) preps were prepared using an osmotic shock to extract the scFv's from the periplasmic space of the bacteria E. *coli* SF110f' according to Marks et al. (1991). After extraction, the scFv preps (TES preps) were then dialyzed three times at 4°C against 5 liter of PBS for 4 hrs. Cryosections of a Human Grawitz tumor and a healthy part of the same kidney were placed on a Silan (Sigma) coated glass slide and dried overnight at room temperature. The cryosections were re-hydrated in PBS and blocked with PBS 4% BSA (ICN) for 20 min. The sections were then incubated with undiluted scFv preps, that were prepared as described supra or EN4 monoclonal antibody (Monosan) directed against CD31 (diluted 1:200) in PBS + 1% BSA for 45 min as a positive control for endothelial cells staining. Then, sections were washed 3 times in PBS/0.05% Tween20 for 5 min and incubated with either culture medium from the 9E10 cell line (containing the anti Myc epitope antibody (Boehringer Mannhein) or a Rabbit Anti-Mouse antiserum (RAMPO, DAKO, diluted 1:250 in PBS + 1% BSA) for 30 min. After 3 times washing in PBS/0.05% Tween20, the scFv stainings were incubated with RAMPO and the EN4 sections were incubated with a Swine Anti-Rabbit antiserum (SWARPO, DAKO, diluted 1:250 in PBS + 1% BSA) for 30 min. Subsequently, the sections with the EN4 staining were washed 3 times with PBS/0.05% Tween20 for 5 min and 3 times with PBS for 5 min. The sections with the scFv staining were washed 3 times with PBS/0.05% Tween20 for 5 min and incubated with SWARPO for 30 min. After this SWARPO incubation the sections were again washed 3 times in PBS/0.05% Tween20 for 5 min and 3 times in PBS. The staining following these procedures was performed with DAB tablets dissolved in PBS (Sigma) and all sections were counter-stained with heamatoxiline (Merck) for 30 sec. The sections were imbedded using glycergel (DAKO) and analyzed using a light microscope.

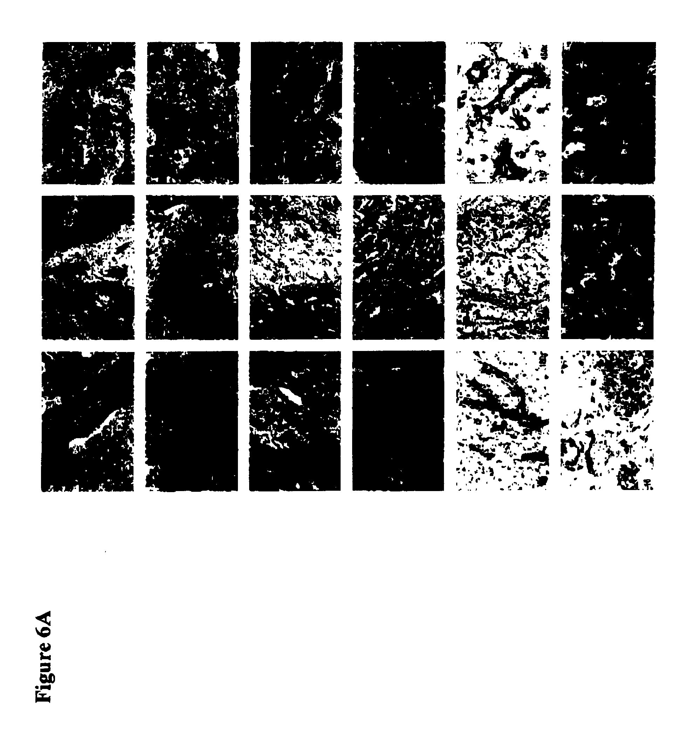

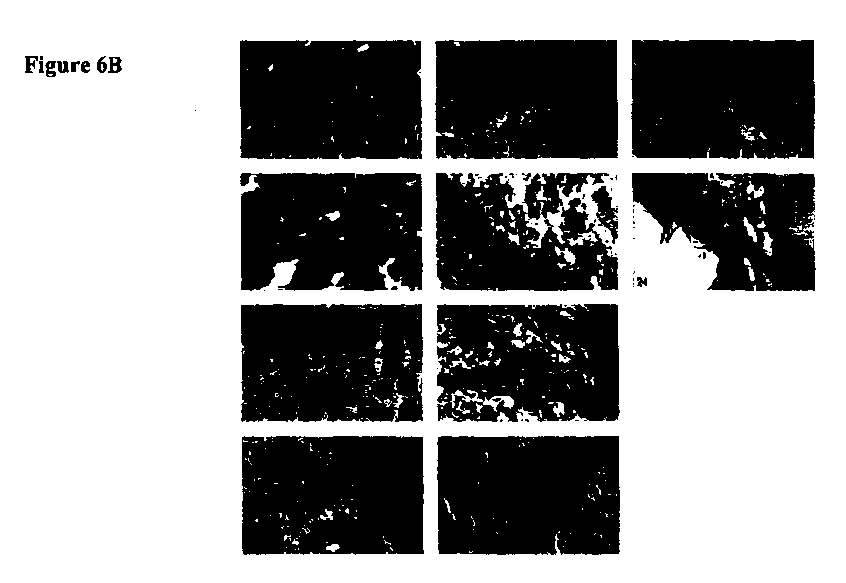

Other tumor tissues that were analyzed using the same protocol as described for the healthy kidney tissue and the Gravitz tumor cells included: Breast Carcinoma, Prostate Carcinoma, Colon Carcinoma, Lung tumor, Melanoma cells, Pancreas tumor, Bladder tumor and Head and Neck tumor cells. Moreover, from the same tissues healthy cells were also tested for FibMab reactivity, except for healthy lung tissues. All of the healthy tissues showed no staining with FibMab. Fig.6A and B show immuno-histochemical staining of either scFv FibMab or an anti-CD31 scFv (EN4) on different human tumor tissues and healthy human tissues, which were:
#1-5 lungcarcinoma with FibMab (positive area)
#6 lungcarcinoma with FibMab (negative area)
#7-9 mama carcinoma with FibMab
#10-11 melanoma with FibMab (positive area)
#12 melanoma with FibMab (negative area)
#13-15 Grawitz kidney tumor with FibMab
#16 healthy kidney tissue with FibMab
#17 transplantation kidney with FibMab
#18 transplantation kidney with anti-CD31
#19 healthy pancreas with FibMab
#20 pancreas tumor with FibMab
#21 bladder tumor with FibMab
#22-24 head and neck tumor with FibMab
#25 healthy colon with FibMab
#26 colon carcinoma with FibMab
#27 prostate tumor with FibMab
#28 healthy prostate with FibMab

In pictures numbers 1 to 5 of figure 6, a lung carcinoma is stained with scFv Fibmab. Clearly visible is the presence of separate positive and negative areas in the tumor tissue. Picture 2 shows a distinct stromal staining in the tumor and no clear endothelial cell or vessel/capillary staining can be observed. Picture 3 shows a large area of tumor stroma that is negative for Fibmab, indicating that Fibmab only recognizes a portion of the tumor stroma in lung carcinoma tissue. Picture 4 and 5 show a positive area at a larger magnification. Pictures 7 and 8 show positive staining on a mamma carcinoma in which a vascular or capillary structure can be recognized but also a stromal staining is present. Picture 9 shows a negative area in the tumor tissue again indicating that only a part of the tumor is recognized by scFv Fibmab. In Pictures number 10, 11 and 12 a melanoma tumor is stained with scFv Fibmab and picture 12 shows a relatively "normal and healthy" part of the tumor that also is not recognized by scFv Fibmab. Again a clear stroma staining can be seen in this melanoma tissue. Pictures 13, 14 and 15 show a staining with scFv Fibmab on a Grawitz (kidney) tumor at different magnifications. Clearly visible is a vascular or capillary staining in this tissue. The tumor is highly vasculerized. 16 and 17 are stainings with Fibmab on transplantation kidney and this is regarded as healthy kidney. No staining can be found in healthy kidney tissue. Picture 18 shows a positive endothelial staining with monoclonal antibody EN4 (recognizing CD31) on transplantation kidney and clearly visible are the vessels in the tubular area and positive staining of the capillaries in the glomerulus of the kidney. Picture 19 showed a healthy pancreas stained with scFv Fibmab and no staining can be seen whereas the pancreas tumor in picture 20 shows a clear stromal staining with scFv Fibmab. Picture 21 shows a positive staining on a bladder tumor in which capillary or vessel structures can be seen. Pictures 22 and 24 show a clear positive staining in a head and neck tumor with scFv Fibmab and picture 22 shows a relatively healthy portion of the same tissue which does not react with the scFv Fibmab. No staining with scFv Fibmab can be found in healthy colon (picture 25) but a colon carcinoma reacts strongly with the scFv Fibmab in the tumor stroma of this tissue. Picture 27 depicts a positive stromal and vessel like staining with scFv Fibmab in a prostate tumor and the healthy tissue control is negative for binding the scFv Fibmab.

In conclusion, scFv Fibmab does only react with all the tumor tissue tested sofar and does not show any reactivity with the healthy tissue controls. It appears that the scFv Fibmab recognizes either a stromal factor in all these tumors but also positive staining can be found in the vasculature in parts some of these tumors.

### Example 6: The use of FibMab in immuno-precipitation experiments to analyze the recognized antigen.

scFv TES preps (prepared as described supra) from the His-Myc construct were used for immuno-precipitation. Immunoprecipitations were performed on the plasma cell line RPMI 8226, that stained positive for FibMab (Table I) on FACS analysis. Cells were cultured in RPMI 1640 medium + 25 mM Hepes, L-glutamine and 5% Fetal Calf Serum (FCS). RPMI 8226 is a semi-adherent plasma cell line and cells do detach from the plates easily by shaking gently. B-cells were obtained from tonsil patient material and prepared according to Van der Vuurst de Vries et al. (1999a) and served as a negative control cell line in these experiments for FibMab. scFv's that were used were: FibMab and P9 (scFv's that recognizes endothelial cells but with a different expression pattern, isolated according to the same selection procedure) on RPMI 8226 cells, III-1 (twice) and I-1 (B-cell specific scFv's, Van der Vuurst de Vries et al. 1999b) were used as a positive control for immuno-precipitation procedures on tonsil B-cells (Van der Vuurst de Vries et al. 1999b), Tg and DNP are scFv's that were selected against peptides according to De Kruif et al. (1995) and served as negative controls on RPMI 8226 cells and as preclearing scFv respectively. III-1 was also used on RPMI 8226 cells where it served as a negative control for precipitated proteins. Pre-clearing scFv's were dialyzed against PBS as described supra. All scFv's were coupled to chelating sepharose beads (Amersham).

### Preparation of Sepharose beads

Sepharose beads were prepared and coupled to the scFv's as follows. 500 µl Sepharose beads were washed 2 times with 1 ml bidest water, followed by 2 times with 1 ml 50 mM EDTA, 1 time with 2 ml 100 mM CoCl₂, 1 time with 2 ml bidest water and 2 times with 1 ml PBS. The beads were then incubated with 10 ml scFv prep by rocking for 30 min on a rocking platform and then washed two times with 4 ml PBS. These scFv bound beads were incubated with PBS + 0.03% H₂O₂ while rocking for 2 hrs at room temperature and subsequently washed with 2 times 2 ml PBS. Beads were then washed once with 2 ml PBS + 50 mM EDTA and washed two times with 2 ml PBS. Beads were then washed with 2 ml PBS + 0.5 M Imidazole and again washed 2 times with 2 ml PBS and stored at 4°C till further use, which was typically within 24 hrs.

### Preparation of cells

HDMEC-1 cells were grown as described supra. For each separate immuno-precipitation 2x10⁶ B-cells were used and all steps were performed at 4°C. Cells were washed 3 times with 20 ml PBS and subsequently resuspended in 1 ml PBS. 10 µl Normal Human Serum (NHS) sulpho-biotin (200 mg/ml in DMSO) was added to the cells and incubated for 30 min with occasionally gentle stirring. Then 10 µl 1 M NH₄Cl in PBS was added to the cells and washed twice with 2 ml 10 mM NH₄Cl. Cells were again washed twice with PBS and then lysed in 1 ml Lysis buffer (1% Triton X100) plus protease inhibitors (Cocktail tablets, Boehringer Mannheim).

### Immunoprecipitations

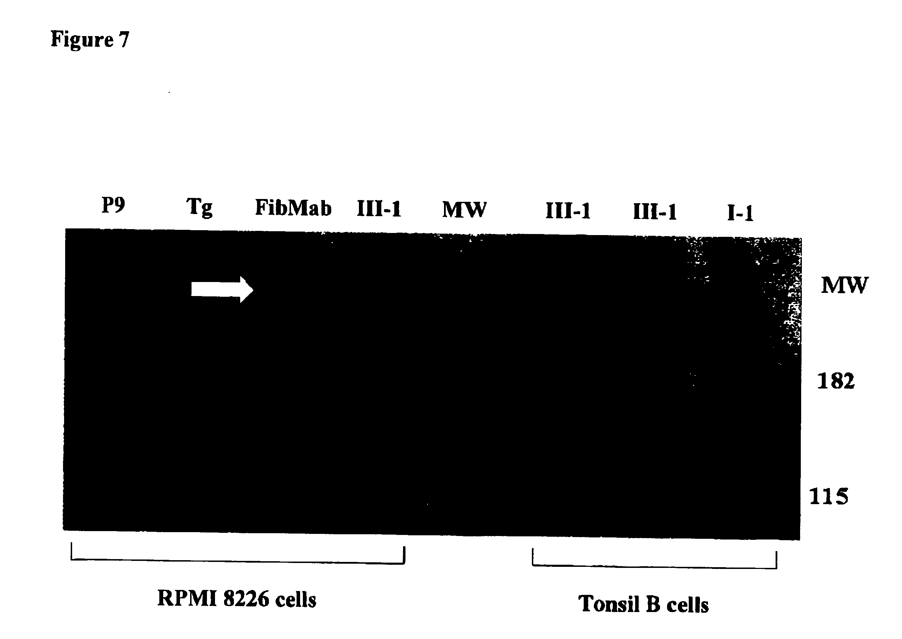

The cell lysates were spun for 1 min at 14,000 rpm and pre-cleared 3 times for 1 hr at 4°C with 100 µl beads coupled to scFv DNP. These pre-cleared lysates were divided over the different immuno-precipitations and incubated with 40 µl beads each at 4°C while rocking overnight. Then, the beads were washed 10 times for 10 min with lysis and 10 times 10 min with PBS. Beads were resuspended in 40 µl PBS and 4 µl 10X Laemmli sample buffer was added. Samples were boiled for 1 min and separated using a 7%-SDS/PAGE gel. Separated proteins were then blotted onto a PVDF membrane (Boehringer Mannheim) and blocked overnight in 2% gelatine. The membrane was then incubated for 30 min with streptavidin HRP (Amersham) and washed 10 times for 5 min in PBS/0.1% Tween20 and washed 3 times for 5 min with PBS. For development of the blot Chemoluminescence blotting substrate (Boehringer Mannheim) was used following the instructions of the manufacturer and Hyperfilm (Hyperfilm MP) was used for detection of the produced signal. Fig.7 Shows the blot of the SDS/Page separated immuno-precipitations of the different scFv's on tonsil B-cells and the plasma cell line RPMI 8226. Bound biotinylated membrane proteins were visualized using streptavidine HRP in combination with Supersignal (Pierce). The result shows the positive signal on tonsil B-cells for scFv's III-1 and I-1 as was expected. scFv FibMab shows a specific band of a high molecular weight and P9 does not precipitate any proteins at the used conditions. Controls I-1 and Tg scored negative on tonsil B cells and RPMI 8226 cells respectively, as expected.

### Protein sequencing

To obtain sufficient amounts of antigen that is recognized by FibMab the whole procedure was scaled up 10 times and the specific FibMab IP band was excised from the SDS/PAGE gel and stored on ice. This was send to TOPLAB (Hamburg, Germany) where some amino acid sequences within the recognized antigen were determined. Three peptide stretches that were sequenced were identified as being a fragment of the fibronectin protein. These identified peptides were:
1) AFTDVDVD
2) IPGHLNSYTIK
3) SSPVTGYRVT
After analysis using the NCBI database, it was found that all peptide tags showed homology with the fibronectin precursor, which is the unspliced fibronectin. Peptide tag 1) showed homology with the ED-A fibronectin splice variant.

### Example 7: Cloning procedures to obtain a fully human IgG1 monoclonal antibody from the FibMab scFv.

The procedures that were followed for the cloning of fully human IgG1 monoclonal antibodies were described by Boel et al. (2000).

### Primers

1. HAVT+HindIII: 5'-AAG CTT CCC ATA AGG AAT TCG GAT CCC
ACG ATG GCA TGC CCT GG-3'
2. M13: 5'-GT AAA ACG ACG GCC AGT-3'
3. Vκ1a : 5'-TGT GAC ATC GAG CTC ACC CAG TCT CCA TCC-3'
4. Jκpan: 5'-TTC TCG ACT TGC GGC CGC AAA GTG CAC TTA CGT
TTG
ATC TCC ACC TTG GTC CC-3'

### Cloning of the Vh fragment

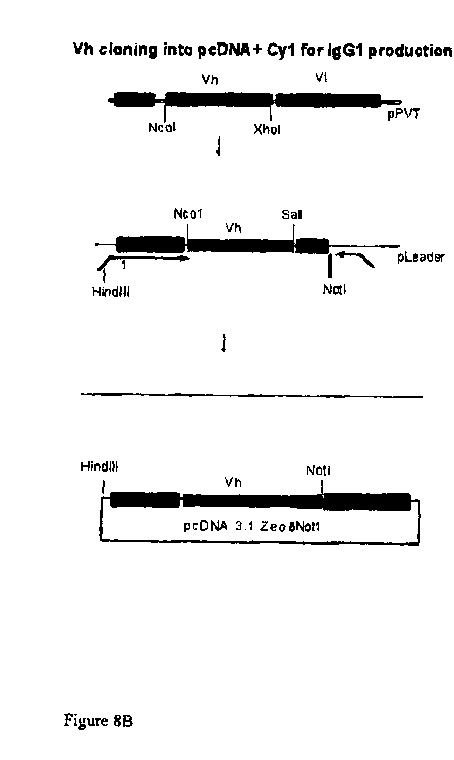

The Vh fragment was excised from the pPVT vector containing the genes encoding the scFv FibMab with NcoI and XhoI restriction enzymes, purified over gel and ligated into pLeader, which is a vector that contains a Kozak sequence and a donor splice site that was digested with NcoI and SalI and purified over gel. This resulted in a plasmid named pLeader VhFibMab. The cloned insert was subsequently amplified by PCR: 96°C for 5 min followed by 28 times 1)96°C for 30 sec, 2) 58°C for 30 sec, 3) 72°C for 1 min and finalized by 72°C for 7 min. The reaction product was then cooled to 4°C. Primers that were used for the PCR were M13 and "HAVT+HindIII" for introduction of a HindIII site at the 5'-end. Furthermore, the HAVT20 leader sequence was altered slightly by changing the translation start sequence into 5'-CCACGATGG-3'. pcDNA 3.1 ZEO (Promega) was mutated by eliminating the NotI in the multiple cloning site (pcDNA 3.1 δNotI ZEO) because the NotI could interfere with cloning further downstream. This mutation was introduced by digesting pcDNA Zeo with NotI and subsequently by filling in the sticky ends using Klenow enzyme and free nucleotides. The constant heavy region of a human IgG (Cγ1) fragment with the Vh of another scFv (UBS-54 directed against human Ep-Cam) was inserted using the BamHI and EcoRV restriction sites. The PCR product of the Vh FibMab was then swapped with HindIII and NotI into pcDNA 3.1 ZEO δNotI + Cγ1. Fig.8B shows the schematic representation of the cloning procedures described for Vh.

### Cloning of the Vl fragment

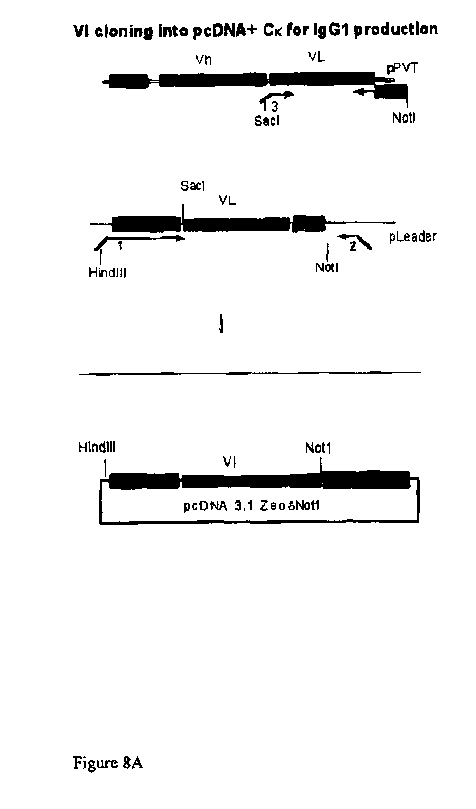

The Vl fragment was amplified by PCR by using the same PCR protocol as described in the cloning of the Vh fragment from the pPVT vector with primers "Vκ1a" and "Jκpan". In this step, the NotI site was deleted and reintroduced at the 3' site from the donor splice site, which was also introduced by primer Jκpan. The PCR product was digested with SacI and NotI and cloned into the pLeader vector which is a vector that contains a Kozak sequence and a donor splice site with SacI and NotI resulting in pLeader VlFibMab. This fragment was again amplified with primers "HAVT+HindIII" and "M13" for the same purpose as in the Vh cloning to introduction a HindIII site at the 5'-end. Furthermore, the HAVT20 leader sequence was altered slightly by changing the translation start sequence into 5'-CCACGATGG-3'._The Vl of a different scFv (UBS-54) with the constant region of the light chain of a IgG1 (Cκ) was cloned into pcDNA 3.1 δNotI ZEO using BamH1 and EcoR1. The Vl of FibMab was then swapped using HindIII and NotI. Fig.8A shows the schematic representation of the cloning procedures described for Vl.
The same procedure is followed when cloning the scFv FibMab into vectors that can produce human IgG2, IgG3, IgG4, IgA1, IgA2, IgM and IgE. These different antibodies and possible fusion proteins are used in in vivo, ex-vivo and in vitro tumor models to test there capacity to block or inhibit angiogenesis, matrix formation and reduction or possible elimination of tumors.

### Short description of the figures

Fig.1. FACS-staining of HDMEC-1 cells incubated with FibMab. The Thyro scFv is the same as Tg in example 5, identified by De Kruif et al. (1995) and served as a negative control. P9 is a scFv that was identified along with the FibMab scFv, which served as a positive control for staining.

Fig.2. FACS-staining of FibMab on RPMI 8226 cells.

Fig.3. FACS-staining of FibMab on Peripheral Blood Leucocytes (PBL's). Identification of B-cells by double staining PBL's with mouse anti CD20 (CLB, Amsterdam) to identify the B-cell population

Fig.4. FACS-staining of FibMab on PBL's on the monocyte compartment.

Fig.5. FACS-staining of FibMab on PBL's on the granulocyte compartment.

Fig.6A. Immuno-histochemical staining of scFv FibMab on different human tumor tissues and healthy human tissues (see text for details).

Fig.6B Immuno-histochemical staining of scFv FibMab on different human tumor tissues and healthy human tissues (see text for details).

Fig.7. Proteins immunoprecipitated by the FibMab scFv and a number of control scFv's on RPMI 8226 cells and tonsil B cells as indicated in the text. The molecular weight of the proteins (MW) is given on the right. The arrow points to the high molecular weight band that is immuno-precipitated specifically by the FibMab scFv.

Fig.8A. Schematic representation of the cloning of the Vl FibMab vector.

Fig.8B. Schematic representation of the cloning of the Vh FibMab vector.

### References

Ades EW, Candel FJ, Swerlick RA, George VG, Summer S, Bosse DC, Lawley TJ. 1992. HDMEC-1: establishment of an immotalized human microvascular endothelial cell line. J. Invest. Dermatol. 99:683-690
Boel E, Verlaan S, Poppelier MJ, Westerdaal NA, Van Strijp JA, Logtenberg T. (2000) Functional human monoclonal antibodies of all isotypes constructed from phage display library-derived single-chain Fv antibody fragments. J Immunol Methods. 26;239: 153-166
Brooks PC, Clark RA, Cheresh DA. (1994) Requirement of vascular integrin alpha v beta 3 for angiogenesis. Science 264:569-571
De Kruif J, Boel E, Logtenberg T. (1995) Selection and application of human single chain Fv antibody fragments from a semi-synthetic phage antibody display library with designed CDR3 regions. J Mol Biol 248:97-105
Friedlander M, Brooks PC, Shaffer RW, Kincaid CM, Varner JA, Cheresh DA. (1995) Definition of two angiogenic pathways by distinct alpha v integrins. Science 270:1500-1502
Ignar DM, Andrews JL, Witherspoon SM, Leray JD, Clay WC, Kilpatrick K, Onori J, Kost T, Emerson DL. (1998) Inhibition of establishment of primary and micrometastatic tumors by a urokinase plasminogen activator receptor antagonist. Clin Exp Metastasis. 16:9-20
Jankun J, Keck RW, Skrzypczak-Jankun E, Swiercz R. (1997) Inhibitors of urokinase reduce size of prostate cancer xenografts in severe combined immunodeficient mice. Cancer Res. 57:559-63.
Köhler G and Milstein C (1975) Continuous cultures of fused cells secreting antibody of predefined specificity. Nature 256:495
Liotta LA, Steeg PS, Stetler-Stevenson WG. (1991) Cancer metastasis and angiogenesis: an imbalance of positive and negative regulation. Cell 64:327-336
Marks JD, Hoogenboom HR, Bonnert TP, McCafferty J, Griffiths AD, Winter G. 1991. By-passing immunization. Human antibodies from V-gene libraries displayed on phage. J. Mol. Biol. 222:581-597
Min HY, Doyle LV, Vitt CR, Zandonella CL, Stratton-Thomas JR, Shuman MA, Rosenberg S. (1996) Urokinase receptor antagonists inhibit angiogenesis and primary tumor growth in syngeneic mice. Cancer Res. 56:2428-2433
Pujuguet P, Hammann A, Moutet M, Samuel, JL, Martin F, Martin M. 1996. Expression of fibronectin ED-A⁺ and ED-B⁺ isoforms by human and experimental colorectal cancer. American J. Pathology. 148:579-592
Reza Farnoud M, Farahnaz Farhadian, Samuel JL, Derome P, Peillon F, Yuan Li J (1995) Fibronectin isoforms are differentially expressed in normal and adenomatous human anteriar pituitaries. Int. J. Cancer. 61:27-34
Van der Vuurst de Vries A-R, Clevers H, Logtenberg T, Meyaard L. (1999a). Leucocyte associated immunoglobulin-like receptor-1 (lair-1) is differentially expressed during human B-cell differentiation and inhibits B-cell receptor-mediated signaling. Eur. J. Immunol.29(10):3160-3167
Van der Vuurst de Vries A-R, Logtenberg T. (1999b). Dissecting the human peripheral B-cell compartment with phage display-derived antibodies. Immunology 98(1):55-62
Werb Z. (1997) ECM and cell surface proteolysis: regulating cellular ecology. Cell 91:439-442

## Claims

1. A proteinaceous molecule capable of specifically binding to an epitope present in a subset of fibronectin proteins.

2. A proteinaceous molecule according to claim 1, capable of binding to extracellular matrix located in the vicinity of a cell.

3. A proteinaceous molecule according to claim 1 or claim 2, wherein said cell comprises a tumor cell.

4. A proteinaceous molecule according to any one of claims 1-3, capable of distinguishing a subset of fibronectin comprising cells.

5. A proteinaceous molecule according to anyone of claims 1-4, capable of binding to a neoplastic cell.

6. A proteinaceous molecule according to claim 5, wherein said neoplastic cell is derived from a breast cell, prostate cell, colon cell, lung cell, skin cell, pancreas cell, bladder cell, head cell or a neck cell.

7. A proteinaceous molecule according to any one of claims 1-6, capable of binding to a microvascular endothelial cell.

8. A proteinaceous molecule according to any one of claims 1-7, capable of binding to a subset of human fibronectin proteins.

9. A proteinaceous molecule according to any one of claims 1-8, wherein said molecule is an antibody or a functional part or derivative thereof.

10. A proteinaceous molecule according to any one of claims 1-9, wherein said molecule is a single chain antibody.

11. A proteinaceous molecule according to claim 9 or 10, wherein said antibody is human or humanized.

12. A proteinaceous molecule according to any one of claims 1-11, further comprising a tag.

13. A proteinaceous molecule according to claim 12, wherein said tag comprises a toxin and/or a radioactive substance.

14. A proteinaceous molecule according to any one of claims 1-13, comprising a heavy chain comprising a CDR3 region, said CDR3 region comprising the sequence EDTAVYYCAR NPFQSS FDYWGQ or a derivative and/or functional analogue thereof.

15. A proteinaceous molecule according to any one of claims 1-14, comprising a light chain comprising a CDR3 region, said CDR3 region comprising the sequence EDFATYYC SQFSTMPGGFGQGTK VEIK or a derivative and/or functional analogue thereof.

16. A method for the treatment of an individual suffering from or at risk of suffering from a disease, comprising administering to said individual a therapeutically acceptable amount of a proteinaceous molecule according to any one of claims 1-15.

17. A method according to claim 16, wherein said disease is a neoplastic disease.

18. Use of a proteinaceous molecule according to any one of claims 1-15 for the preparation of a medicament.

19. Use according to claim 18, for the treatment of neoplastic disease.

20. A method for typing a cell comprising determining whether said cell is capable of specifically binding a proteinaceous molecule according to any one of claims 1-15.

21. Use of an epitope expressed on a subset of fibronectin expressing cells as a marker for neoplastic cells.

22. A nucleic acid encoding a proteinaceous molecule according to any one of claims 1-15, or a part thereof involved in fibronectin binding of said molecule.

23. A cell comprising a nucleic acid according to claim 22.

24. A cell according to claim 23, wherein said cell is a primate, rodent, bird or plant cell.

25. A cell according to claim 23 or 24, wherein said cell is a human cell.

26. A cell according to claim any one of claims 23-25, further comprising a means for the conditional expression of a nucleic acid of interest.

27. A cell according to claim 26, wherein said means comprises a tetracycline responsive expression system.

28. A cell according to any one of claims 23-27, wherein said cell comprises nucleic acid encoding an early protein of adenovirus or a functional part, derivative and/or analogue thereof.

29. A cell according to claim 28, wherein said early protein comprises adenovirus E1 or a functional part, derivative and/or analogue thereof.

30. A cell according to claim 28 or 29, comprising adenovirus E2A or a functional part, derivative and/or analogue thereof.

31. A plant or a non-human animal comprising a cell according to any one of claims 23-30.

32. A plant or a non-human animal according to claim 31, transgenic for a nucleic acid according to claim 22.

33. A gene delivery vehicle comprising a proteinaceous molecule according to anyone of claims 1-15.

34. A method for the treatment of an individual suffering from or at risk of suffering from a disease, comprising administering to said individual a therapeutically acceptable amount of a gene delivery vehicle according to claim 33.

35. A method according to claim 34, wherein said disease is a neoplastic disease.

36. Use of a gene delivery vehicle according to claim 33 for the preparation of a medicament.

37. Use according to claim 36, for the treatment of neoplastic disease.

38. A kit comprising a proteinaceous molecule according to anyone of claims 1-15.

39. A kit comprising a gene delivery vehicle according to claim 33.

40. A cell recognized by a proteinaceous molecule according to any one of claims 1-15.

41. A cell recognized by a gene delivery vehicle according to claim 33.
